# FASCICULE DE BREVET EUROPEEN

(11) **EP 0 632 035 B1**
(45) Date de publication et mention de la délivrance du brevet: **09.10.1996**
(21) Numéro de dépôt: 94202873.9
(22) Date de dépôt: 21.07.1993
(51) Int. Cl.: C07D 405/06, A61K 31/445

(54) **Nouvelles aminoalkylchromones, leur procédés de préparation et les compositions pharmaceutiques qui les contiennent**
Aminoalkylchomone, Verfahren für ihre Herstellung und die enthaltende pharmazeutische Zusammensetzungen
Aminoalkylchomones, process for their preparation and pharmaceutical compositions containing them

(30) Priorité: 21.07.1992 FR 9208950
(43) Date de publication de la demande: 04.01.1995
(62) Demande divisionnaire de: 93401876.3
(73) Titulaire: ADIR ET COMPAGNIE, F-92415 Courbevoie Cédex (FR)
(72) Inventeur: Payard, Marc, F-31130 Balma (FR); Baziard-Mouysset, Geneviève, F-31000 Toulouse (FR); De Saqui-Sannes, Gilbert, F-31000 Toulouse (FR); Guardiola, Béatrice, F-92200 Neuilly sur Seine (FR); Caignard, Daniel-Henri, F-75015 Paris (FR); Renard, Pierre, F-78000 Versailles (FR); Adam, Gérard, F-78600 Le Mesnil le Roi (FR)

(56) Documents cités:
- EP-A- 0 428 437
- EP-A- 0 457 686
- CHEMICAL ABSTRACTS, vol. 90, no. 19, 7 Mai 1979, Columbus, Ohio, US; abstract no. 145542w,
- CHEMICAL ABSTRACTS, vol. 106, no. 11, 16 Mars 1987, Columbus, Ohio, US; abstract no. 78636p,

## Description

La présente invention concerne de nouvelles aminoalkylchromones, leurs procédés de préparation et les compositions pharmaceutiques qui les contiennent.

Certaines aminoalkylchromones sont déjà connues (brevet US 3,767,679) pour leur activité dans la prévention des manifestations allergiques.

L'art antérieur le plus proche, brevet EP-A-428437, décrit des pipéridin-4-ylalkylbenzisoxazoles possédant des activités antagonistes vis-à-vis de la dopamine et de la serotonine.

La demanderesse a présentement découvert de nouvelles aminoalkylchromones qui possèdent de remarquables propriétés pharmacologiques.

Les composés de l'invention se révèlent notamment être de puissants ligands des récepteurs sigma (σ) et à ce titre peuvent être utilisés dans les troubles du système nerveux central, ce qui les distingue totalement des composés de l'art antérieur.

Plus particulièrement, la présente invention concerne les composés de formule (I) : 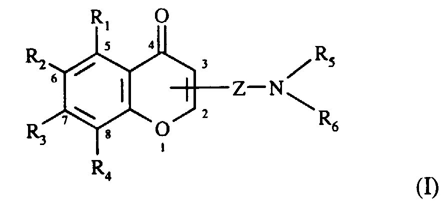 dans laquelle :
- Z: représente un chaînon méthylène éventuellement substitué par un radical acyle linéaire ou ramifié et comportant de 1 à 6 atomes de carbone,
- R₁, R₂, R₃, R₄: représentent, indépendamment les uns des autres, un radical choisi parmi :
- hydrogène,
- halogène,
- hydroxy,
- alkoxy linéaire ou ramifié et comportant de 1 à 6 atomes de carbone,
- alkyle linéaire ou ramifié et comportant de 1 à 6 atomes de carbone,
- phényle,
- benzyle,
- trifluorométhyle,
- R₅ et R₆: forment ensemble, avec l'atome d'azote qui les porte une pipéridine substituée par un radical phényle, naphtyle, phénylalkyle linéaire ou ramifié de 1 à 6 atomes de carbone ou naphtylalkyle linéaire ou ramifié de 1 à 6 atomes de carbone, éventuellement substitués sur les noyaux phényle et naphtyle par un ou plusieurs radicaux choisis parmi :
- halogène,
- hydroxy,
- mercapto,
- trifluorométhyle,
- alkyle linéaire ou ramifié comportant de 1 à 6 atomes de carbone,
- alkoxy linéaire ou ramifié comportant de 1 à 6 atomes de carbone,
- et alkoxycarbonyle linéaire ou ramifié comportant de 1 à 6 atomes de carbone,
leurs isomères optiques, ainsi que leurs sels d'addition à un acide ou à une base pharmaceutiquement acceptable.

Parmi les acides utilisés pour la formation des sels d'addition des composés de formule (I), on peut citer, à titre d'exemples et de façon non limitative, dans la série minérale, les acides chlorhydrique, bromhydrique, sulfurique et phosphorique, et dans la série organique, les acides acétique, propionique, maléique, fumarique, tartrique, citrique, oxalique, benzoïque et méthanesulfonique.

Parmi les bases utilisées pour la formation des sels d'addition des composés de formule (I), on peut citer, à titre d'exemples et de façon non limitative, dans la série minérale, les hydroxydes de sodium, de potassium, de calcium et d'aluminium, les carbonates de métaux alcalinoterreux, et dans la série organique, la triéthylamine, benzylamine, diéthylamine, tertiobutylamine, dicyclohexylamine et l' arginine.

La présent invention s'étend également au procédé de préparation des composés de formule (I), caractérisé en ce que l'on fait réagir une amine de formule (II) : 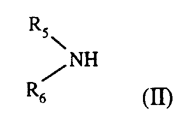 dans laquelle R₅ et R₆ sont tels que définis dans la formule (I),
avec un dérivé de l'halogénoalkyle chromone de formule (III) : 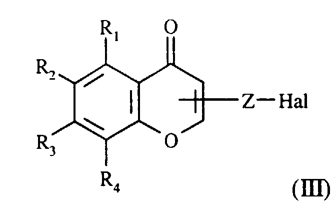 dans laquelle R₁, R₂, R₃, R₄ et Z sont tels que définis dans la formule (I) et Hal représente un atome d'halogène,
afin d'obtenir les composés de formule (I),
composés de formule (I) qui peuvent être, si on le désire,
- purifiés suivant une ou plusieurs méthodes de purification choisies parmi la cristallisation, la chromatographie sur gel de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou une base pharmaceutiquement acceptable.

La présente invention s'étend également au procédé de préparation des composés de formule (I/A) : 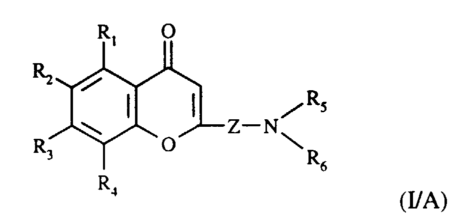 dans laquelle R₁, R₂, R₃, R₄, R₅, R₆ et Z sont tels que définis dans la formule (I),
cas particulier des composés de formule (I) pour lesquels la chromone est substituée en position 2 par le radical 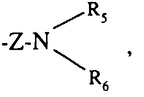 caractérisé en ce que l'on fait réagir une amine de formule (II) : 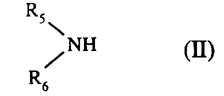 dans laquelle R₅ et R₆ sont tels que définis dans la formule (I), avec un composé de formule (IV) :

Hal'-Z-COO-CH₂CH₃ (IV)

dans laquelle Z est tel que défini précédemment, et Hal' représente un atome d'halogène, afin d'obtenir un composé de formule (V) : 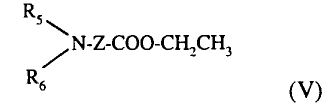 dans laquelle R₅, R₆ et Z sont tels que définis précédemment,
composé de formule (V) que l'on fait réagir avec un dérivé de l'ortho-hydroxy acétophénone de formule (VI) : 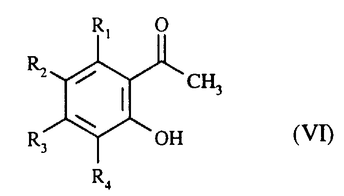 dans laquelle R₁, R₂, R₃ et R₄ sont tels que définis dans la formule (I),
afin d'obtenir les composés de formule (I/A) tels que définis précédemment,
composés de formule (I/A) qui peuvent être, si on le désire,
- purifiés suivant une ou plusieurs méthodes de purification choisies parmi la cristallisation, la chromatographie sur gel de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou une base pharmaceutiquement acceptable.

Les matières premières utilisées dans les procédés précédemment décrits sont :
- soit commerciales,
- soit aisément accessibles à l'homme de métier selon des procédés décrits dans la littérature.

La demanderesse a découvert que les composés de l'invention possédaient des propriétés pharmacologiques très intéressantes.

Des essais de liaison aux récepteurs σ (sigma) ont en effet montré que les composés de l'invention se comportent comme de très puissants ligands de ces récepteurs ("Mesure de l'affinité des composés de l'invention pour les récepteurs σ (sigma)" : exemple B de la présente demande).

Par ailleurs, la demanderesse a découvert que les composés de formule (I) présentent une très bonne sélectivité vis à vis des récepteurs σ (sigma), notamment par rapport aux récepteurs dopaminergiques D₂ ("Mesure de l'affinité des composés de l'invention pour les récepteurs β₁, β₂, D₁, D₂, 5HT_{1A}, 5HT_{1C}, 5HT_{1D}, 5HT₂ et 5HT₃" : exemple C de la présente demande).

Cette sélectivité leur confère de remarquables propriétés neuroleptiques, antipsychotiques et psychotogéniques pures ("Etude de l'antagonisme de l'hyperactivité induite par la d-Amphétamine sulfate chez la souris" : exemple D de la présente demande et "Etude de l'antagonisme des stéréotypes comportementaux induit chez le rat par la phencyclidine" : exemple E de la présente demande), dépourvues des effets secondaires dus à la composante extra-pyramidale couramment associée aux neuroleptiques ("Etude de la réversion des effets de l'apomorphine chez le rat" : exemple F de la présente demande).

Les composés de la présente invention peuvent ainsi être utilisés dans le traitement et la prévention du stress, de l'anxiété, de la dépression, des psychoses et de la schizophrénie, sans avoir à redouter les habituels effets secondaires de type parkinsonien dont l'akinésie, les tremblements, la rigidité des membres, associés aux neuroleptiques de type classique.

La présente invention a également pour objet les compositions pharmaceutiques contenant un dérivé de formule générale (I), ou un de ses sels physiologiquement tolérables, en combinaison avec un ou plusieurs excipients ou véhicules pharmaceutiquement acceptables.

Parmi les compositions pharmaceutiques selon l'invention, on pourra citer, à titre d'exemples et de façon non limitative, celles qui conviennent à une administration par voie orale, nasale ou parentérale et notamment les comprimés, les dragées, les gélules, les paquets, les sachets, les granules, les pilules, les granulés, les suppositoires, les crèmes, les pommades, les aérosols, les capsules, les gels dermiques et les solutions injectables ou buvables.

La posologie varie d'un individu à l'autre, selon l'âge, le poids et le sexe du malade, la voie d'administration choisie, la nature et l'intensité de l'affection. Les doses utilisées s'échelonnent entre 0,1 et 100 mg pour un traitement, divisibles en 1 à 3 prises par 24 heures.

Les exemples suivants illustrent l'invention et ne la limitent en aucune façon.

### EXEMPLE DE REFERENCE 1 :

### 2-[(4-BENZYLPIPERAZIN-1-YL)METHYL]-4-OXO[4H]-1-BENZOPYRANE

### 1er procédé de synthèse

On dissout, dans 200 cm³ de tétrahydrofurane, 0,02 mole de 2-chlorométhyl-4-oxo-[4H]-1-benzopyrane. On ajoute ensuite 0,04 mole de 1-benzylpipérazine. L'évolution de la réaction est suivie par chromatographie sur couche mince. Lorsque tout le dérivé chloré a réagi, on évapore le solvant sous pression réduite. Le résidu est repris par de l'éther. La solution est filtrée, évaporée et chromatographiée sur colonne de gel de silice avec le 1,2-dichloroéthane comme éluant. On obtient le 2-[(4-benzylpipérazin-1-yl)méthyl]-4-oxo[4H]-1-benzopyrane.
Point de fusion (base) : huile
Point de fusion (dichlorohydrate) : 238°C
Rendement (dichlorohydrate) : 79 %

| Caractéristiques spectrales (base) : | | |
|---|---|---|
| Infrarouge : | ν C-H, CH₂ : | 2815, 2879, 2913, 2937, 3026, 3061, 3083 cm⁻¹ |
| | ν C=O : | 1654 cm⁻¹ |
| | ν C=C : | 1463, 1570, 1607 cm⁻¹ |

| | | | |
|---|---|---|---|
| RMN (CDCl₃) | | | |
| δ (ppm) | | | |
| 2,63 | m | 8H | CH₂-N |
| 3,48 | s | 2H | Ar-CH₂-N |
| 3,52 | s | 2H | Ar-CH₂-N |
| 6,43 | s | 1H | H₃ |
| 7,27-7,77 | m | 8H | aromatiques |
| 8,2 | dd | 1H | H₅ |

### EXEMPLE DE REFERENCE 2 :

### 2-[(4-BENZYLPIPERAZIN-1-YL)METHYL]-4-OXO[4H]-1-BENZOPYRANE

### Second procédé de synthèse

### Stade A : 2-(4-benzylpipérazin-1-yl)acétate d'éthyle

Dans un erlen rodé de 500 cm³, on introduit 21 g (0,12 mole) de l-benzylpipérazine, 13,4 cm³ soit 20 g (0,12 mole) de bromoacétate d'éthyle et 200 cm³ d'éthanol. Le mélange est porté 30 minutes à reflux. On ajoute alors 17 g (0,12 mole) de carbonate de potassium et le reflux est poursuivi encore pendant une heure. Après refroidissement, le mélange est filtré et le filtrat est évaporé sous pression réduite. Le résidu est repris par un mélange de 200 cm³ d'eau et 200 cm³ de chloroforme. Après extraction, la phase organique est séchée, filtrée et évaporée.
On obtient ainsi 29 g de 2-(4-benzylpipérazin-1-yl)acétate d'éthyle sous forme d'une huile brute brune.
Cette huile est purifiée par distillation sous pression réduite. On obtient ainsi 22,5 g d'une huile visqueuse incolore.
Point de fusion (base) : huile
Rendement (base) : 72%

| Caractéristiques spectrales (base) : | | |
|---|---|---|
| Infrarouge : | ν CH, CH₂ : | 2811, 2934, 2979, 3025 cm⁻¹ |
| | ν COO : | 1747 cm⁻¹ |
| | ν C=C : | 1451, 1676 cm⁻¹ |

| | | | |
|---|---|---|---|
| RMN (CDCl₃) | | | |
| δ (ppm) | | | |
| 1,2 | t | 3H | CH₃ |
| 2,56 | m | 8H | CH₃ |
| 3,13 | s | 2H | CH₂-CO |
| 3,46 | s | 2H | CH₂-aromatique |
| 4,13 | q | 2H | CH₂O |
| 7,2 | m | 5H | aromatique |

### Stade B : 2-[(4-benzylpipérazin-1-yl)méthyl]-4-oxo[4H]-1-benzopyrane

Dans un erlen de 500 cm³, on introduit 100 cm³ de pyridine anhydre, 13 g (0,05 mole) du composé obtenu au stade A et 7,5 g (0,055 mole) de 2-hydroxyacétophénone. On ajoute ensuite très lentement sous agitation et par petites portions 10 g (0,41 mole) d'hydrure de sodium à 60 % dispersé dans l'huile minérale. Lorsque le dégagement gazeux a cessé, le mélange est porté à reflux et agité pendant 45 minutes. Après refroidissement, on filtre. Le précipité obtenu est lavé à l'éther puis ajouté par petites portions à 50 cm³ d'acide chlorhydrique concentré et refroidi extérieurement par de la glace. La réaction est violente. Lorsque l'addition est terminée, on chauffe à reflux pendant 5 minutes. Après refroidissement, on ajoute 200 cm³ d'eau et on extrait avec deux fois 100 cm³ de chloroforme. Cette dernière phase organique est séchée, filtrée et évaporée. L'huile obtenue est purifiée par chromatographie sur gel de silice en utilisant le méthyl chloroforme comme éluant. On récupère ainsi 6 g de 2-[(4-benzylpipérazin-1-yl)méthyl]-4-oxo[4H]-1-benzopyran.
Point de fusion (base) : huile
Point de fusion (dichlorhydrate) : 238°C

| Caractéristiques spectrales (base) : | | |
|---|---|---|
| Infrarouge : | ν CH, CH₂ : | 2815, 2879, 2913, 2937, 3026, 3061, 3083 cm⁻¹ |
| | ν C=O : | 1654 cm⁻¹ |
| | ν C=C : | 1463, 1570, 1607 cm⁻¹ |

| | | | |
|---|---|---|---|
| RMN (CDCl₃) | | | |
| δ (ppm) | | | |
| 2,63 | m | 8H | CH₂-N |
| 3,48 | s | 2H | Aromatique-CH₂-N |
| 3,52 | s | 2H | Aromatique-CH₂-N |
| 6,43 | s | 1H | H₃ |
| 7,27-7,77 | m | 8H | aromatiques |
| 8,2 | dd | 1H | H₅ |

De la même façon, on obtient :

### EXEMPLE 1:

### 2-{[4-(2-NAPHT-2-YLETHYL)PIPERIDIN-1-YL]METHYL}-4-OXO[4H]-1-BENZOPYRANE

### EXEMPLE 2:

### 2-[(4-PHENYLPIPERIDIN-1-YL)METHYL]-4-OXO[4H]-1-BENZOPYRANE

### EXEMPLE 3:

### 2-[(4-BENZYLPIPERIDIN-1-YL)METHYL]-4-OXO[4H]-1-BENZOPYRANE

Point de fusion (base) : huile
Point de fusion (chlorhydrate) : 208°C
Rendement (base) : 10%
Rendement (chlorhydrate) : 90%

### ETUDE PHARMACOLOGIQUE DES COMPOSES DE L'INVENTION

### EXEMPLE A : ETUDE DE LA TOXICITE DES COMPOSES DE L'INVENTION

### PROTOCOLE :

La toxicité aiguë a été appréciée après administration orale à des lots de 5 souris (20 ± 2 grammes) de doses croissantes (0,1 - 0,25 - 0,50 - 0,75 - 1 g/kg) des composés de l'invention. Les animaux ont été observés à intervalles réguliers au cours de la première journée et quotidiennement pendant les 2 semaines suivant le traitement.

### RESULTATS :

Il apparaît que les composés de l'invention sont atoxiques. Aucun décès n'est observé après administration d'une dose de 1 g.kg-1. On ne constate pas de troubles après administration de cette dose.

### EXEMPLE B : MESURE DE L'AFFINITE DES COMPOSES DE L'INVENTION POUR LES RECEPTEURS σ (SIGMA)

### PROTOCOLE :

L'affinité in vitro des composés de l'invention, pour les récepteurs _, a été déterminée :
- par la mesure du déplacement du ³H-PPP ([3H]-(3-hydroxyphényl)-N-(1-propyl)pipéridine), ligand spécifique du récepteur σ, sur des préparations de cortex cérébral de cobaye,
- et par la mesure du déplacement du ³H-DTG (1,3-Di-(2-tolyl)guanidine), sur des préparations de cerveau de cochon d'Inde.

### RESULTATS :

Les composés de l'invention se révèlent être de très puissants ligands des récepteurs _ (sigma), avec des constantes d'affinité de l'ordre du nanomolaire.

### EXEMPLE C : MESURE DE L'AFFINITE DES COMPOSES DE L'INVENTION POUR LES RECEPTEURS β₁, β₂, D₁, D₂, 5HT_{1A}, 5HT_{1C}, 5HT_{1D}, 5HT₂ET 5HT₃.

### PROTOCOLE :

L'affinité in-vitro des composés de l'invention a été déterminée :
- pour les récepteurs adrénergiques β₁, par la mesure du déplacement du dihydroalprénolol, sur des préparations de cortex frontal de rat,
- pour les récepteurs adrénergiques β₂, par la mesure du déplacement du dihydroalprénolol, sur des préparations de parenchyme pulmonaire de rat,
- pour les récepteurs dopaminergiques D₁, par la mesure du déplacement du SCH 23390, sur des préparations de striatum de rat,
- pour les récepteurs dopaminergiques D₂, par la mesure du déplacement du Raclopride, sur des préparations de striatum de rat,
- pour les récepteurs sérotoninergiques 5HT_{1A}, par la mesure du déplacement du 8-OH-DPAT (8-hydroxy-2-(di-n-propylamino)tétraline), sur des préparations d'hippocampes de rat,
- pour les récepteurs sérotoninergiques 5HT_{1C}, par la mesure du déplacement du N-méthylmésulergine, sur des préparations de cortex frontal et d'hippocampe de rat,
- pour les récepteurs sérotoninergiques 5HT_{1D}, par la mesure du déplacement de la 5-hydroxytryptamine, sur des préparations de cortex, striatum et globus pallidus de rat,
- pour les récepteurs sérotoninergiques 5HT₂, par la mesure du déplacement de l'amino-iodokétansérine, sur des préparations de cortex frontal de rat,
- pour les récepteurs sérotoninergiques 5HT₃, par la mesure du déplacement du BRL 43694, sur des préparations d'aéra postréma de rat.

### RESULTATS :

Les composés de l'invention présentent une affinité pour les récepteurs β₁, β₂, D₁, D₂, 5HT_{1A}, 5HT_{1C}, 5HT_{1D}, 5HT₂ ET 5HT₃ nettement plus faible que pour les récepteurs σ (sigma).

### EXEMPLE D : ETUDE DE L'ANTAGONISME DE L'HYPERACTIVITE INDUITE PAR LA d-AMPHETAMINE SULFATE CHEZ LA SOURIS

### PRINCIPE :

Ce test a pour but d'évaluer l'activité antagoniste éventuelle des composés de l'invention, sur l'hyperactivité induite par la d-amphétamine sulfate. Cette expérience permet de mettre en évidence l'activité neuroleptique d'un composé.

### PROTOCOLE :

Des souris mâles NMRI-CERJ sont placées, à raison de une par cage, dans des labyrinthes formés de 6 compartiments (25,5 × 20,5 × 9 cm) équipés de cellules photoélectriques.

Le nombre de passages de chaque animal, entre les cellules photoélectriques, est mesuré pendant 30 minutes.

Les produits de l'invention sont testés pour des doses s'échelonnant entre 8 et 32 mg/kg. Ils sont injectés, par voie intrapéritonéale, 1 heure avant le début de l'expérience.

La d-amphétamine sulfate (2 mg/kg) est quant à elle injectée par voie intrapéritonéale 30 minutes avant le début du test.

### RESULTATS :

- La d-amphétamine sulfate, injectée seule 30 minutes avant le début de l'expérience, provoque une augmentation de 113 % du nombre de passages entre les cellules photoélectriques.
- Dès 8 mg/kg, les composés de l'invention antagonisent, de façon importante, l'hyperactivité induite chez la souris par la d-amphétamine sulfate, ce qui met en évidence leur activité neuroleptique.

### EXEMPLE E : ETUDE DE L'ANTAGONISME DES STEREOTYPES COMPORTEMENTAUX INDUITS CHEZ LE RAT PAR LA PHENCYCLIDINE

### PRINCIPE :

La phencyclidine, qui est un ligand des récepteurs σ (sigma) induit chez le rat des stéréotypes comportementaux facilement reconnaissables (balancement de la tête, mouvements incontrôlés des pattes antérieures).

Les neuroleptiques classiques antagonisent les effets de la phencyclidine.

### PROTOCOLE :

On injecte à des rats mâles WISTAR CERJ, 8 mg/kg de phencyclidine, par voie sous-cutanée, puis on les place dans des cages transparentes (20 × 10 × 10 cm), à raison de 1 par cage, et on note l'intensité des stéréotypes observés.
Les produits de l'invention sont injectés par voie intrapéritonéale, à des doses s'échelonnant entre 8 et 32 mg/kg, 30 minutes avant la phencyclidine.

Les observations commencent 10 minutes après l'injection de phencyclidine et l'intensité des stéréotypes comportementaux est évaluée pendant 90 minutes.

### RESULTATS :

Dès 8 mg/kg, les composés de l'invention antagonisent de façon significative les stéréotypes comportementaux induits chez le rat par la phencyclidine.
Ce résultat met en évidence l'activité neuroleptique des composés de l'invention.

### EXEMPLE F : ETUDE DE LA REVERSION DES EFFETS DE L'APOMORPHINE CHEZ LE RAT

### PRINCIPE :

Les composés réversant les effets de l'apomorphine présentent une activité non négligeable, au niveau du système extrapyramidal.
Ce test permet donc de savoir si un composé neuroleptique est dépourvu, ou non, d'activité sur le système extrapyramidal.
Cette activité se traduit par des effets de type parkinsonien, tels que l'akinésie, les tremblements et la rigidité des membres.

### PROTOCOLE :

Les composés de l'invention sont administrés à des rats mâles SPRAGUE-DAWLEY, à des doses variant entre 1 et 16 mg/kg.
L'halopéridol (1 mg/kg) injectée dans les mêmes conditions est utilisée comme produit de référence.
Une solution d'apomorphine (1 mg/kg) est injectée par voie sous-cutanée, 30 minutes plus tard.
Les rats sont aussitôt placés dans des cages transparentes, à raison de 1 animal par cage, et on note l'intensité des stéréotypes observés après l'administration de l'apomorphine, pendant 30 minutes.

### RESULTATS :

Les composés de l'invention, même pour des doses de 32 mg/kg se révèlent inaptes à réverser les effets induits par l'apomorphine chez le rat, alors que l'halopéridol (1 mg/kg) fait totalement disparaître ces effets.
Ce test révèle l'absence d'activité des composés de l'invention sur le système extra-pyramidal. Les composés de l'invention sont donc dépourvus d'effets secondaires de type parkinsonien (akinésie, tremblements et rigidité des membres) de par leur inactivité sur le système extra-pyramidal.
Ils présentent donc des propriétés neuroleptiques et antipsycotiques pures dépourvues des effets secondaires dus à la composante extra-pyramidale couramment associée aux neuroleptiques classiques.

## Revendications

1. Composés de formule (I) : 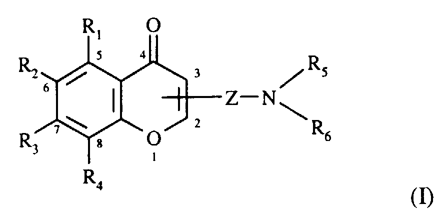 dans laquelle :
Z représente un chaînon méthylène éventuellement substitué par un radical acyle linéaire ou ramifié et comportant de 1 à 6 atomes de carbone,
R₁, R₂, R₃, R₄ représentent, indépendamment les uns des autres, un radical choisi parmi :
- hydrogène,
- halogène,
- hydroxy,
- alkoxy linéaire ou ramifié et comportant de 1 à 6 atomes de carbone,
- alkyle linéaire ou ramifié et comportant de 1 à 6 atomes de carbone,
- phényle,
- benzyle,
- trifluorométhyle,
R₅ et R₆ forment ensemble, avec l'atome d'azote qui les porte une pipéridine substituée par un radical phényle, naphtyle, phénylalkyle linéaire ou ramifié de 1 à 6 atomes de carbone ou naphtylalkyle linéaire ou ramifié del à 6 atomes de carbone, éventuellement substitués sur les noyaux phényle et naphtyle par un ou plusieurs radicaux choisis parmi :
- halogène,
- hydroxy,
- mercapto,
- trifluorométhyle,
- alkyle linéaire ou ramifié comportant de 1 à 6 atomes de carbone,
- alkoxy linéaire ou ramifié comportant de 1 à 6 atomes de carbone,
- et alkoxycarbonyle linéaire ou ramifié comportant de 1 à 6 atomes de carbone,
leurs isomères optiques, ainsi que leurs sels d'addition à un acide ou à une base pharmaceutiquement acceptable.

2. Composés de formule (I) selon la revendication 1 pour lesquels le goupement de formule 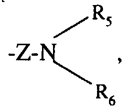 avec Z, R₅, et R₆ tels que définis dans la revendication 1, substitue en position 2 la chromone présente dans la formule (I) selon la revendication 1, leurs isomères optiques,
et leurs sels d'addition à un acide ou à une base pharmaceutiquement acceptable.

3. Composés de formule (I) selon la revendication 1 pour lesquels le groupement de formule 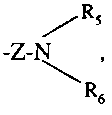 avec Z, R₅, et R₆ tels que définis dans la revendication 1, substitue en position 3 la chromone présente dans la formule (I) selon la revendication 1, leurs isomères optiques,
et leurs sels d'addition à un acide ou à une base pharmaceutiquement acceptable.

4. Composés de formule (I) selon la revendication 1 pour lesquels Z représente un groupement -CH₂-,
leurs isomères optiques,
et leurs sels d'addition à un acide ou à une base pharmaceutiquement acceptable.

5. Composé selon la revendication 1 qui est le 2-[(4-benzylpipéridin-1-yl)méthyl]-4-oxo[4M]-1-benzopyrane, et ses sels d'addition à un acide pharmaceutiquement acceptable.

6. Procédé de préparation des composés de formule (I) selon la revendication 1, caractérisé en ce que l'on fait réagir une amine de formule (II) : 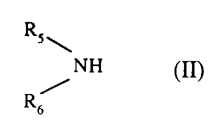 dans laquelle R₅ et R₆ sont tels que définis dans la revendication 1,
avec un dérivé de l'halogénoalkyle chromone de formule (III) : 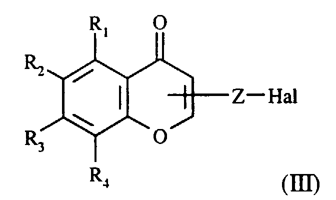 dans laquelle R₁, R₂, R₃, R₄ et Z sont tels que définis dans la revendication 1 et Hal représente un atome d'halogène,
afin d'obtenir les composés de formule (I),
composés de formule (I) qui peuvent être, si on le désire,
- purifiés suivant une ou plusieurs méthodes de purification choisies parmi la cristallisation, la chromatographie sur gel de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou une base pharmaceutiquement acceptable.

7. Procédé de préparation des composés de formule (I/A) : 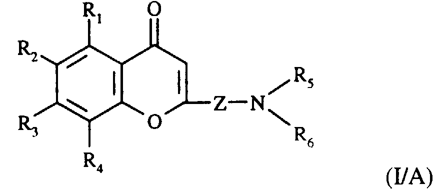 dans laquelle R₁, R₂, R₃, R₄, R₅, R₆ et Z sont tels que définis dans la revendication 1, cas particulier des composés de formule (I), pour lesquels la chromone est substituée en position 2 par le radical : 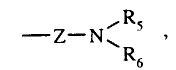 caractérisé en ce que l'on fait réagir une amine de formule (II) : 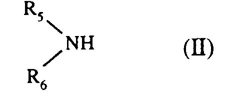 dans laquelle R₅ et R₆ sont tels que définis dans la revendication 1,
avec un composé de formule (IV) :
Hal'-Z-COO-CH₂CH₃ (IV)
dans laquelle Z est tel que défini précédemment, et Hal' représente un atome d'halogène,
afin d'obtenir un composé de formule (V) : 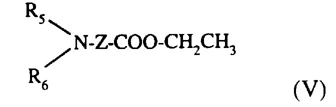 dans laquelle R₅, R₆ et Z sont tels que définis précédemment,
composé de formule (V) que l'on fait réagir avec un dérivé de l'ortho-hydroxy acétophénone de formule (VI) : 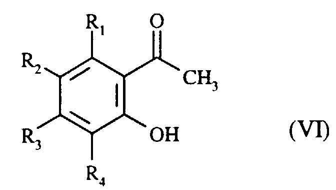 dans laquelle R₁, R₂, R₃ et R₄ sont tels que définis dans la revendication 1,
afin d'obtenir les composés de formule (I/A) tels que définis précédemment,
composés de formule (I/A) qui peuvent être, si on le désire,
- purifiés suivant une ou plusieurs méthodes de purification choisies parmi la cristallisation, la chromatographie sur gel de silice, l'extraction, la filtration, et le passage sur charbon et/ou résine,
- séparés, le cas échéant, sous forme pure ou sous forme de mélange, en leurs éventuels isomères optiques,
- et/ou salifiés par un acide ou une base pharmaceutiquement acceptable.

8. Composition pharmaceutique contenant comme principe actif au moins un composé selon la revendication 1, en combinaison avec un ou plusieurs excipients ou véhicules pharmaceutiquement acceptables.

9. Composition pharmaceutique selon la revendication 8 utile dans le traitement et la prévention du stress, de l'anxiété, de la dépression, des psychoses et de la schizophrénie.

10. Composition pharmaceutique selon la revendication 8 utile dans le traitement et la prévention de la dépression.

11. Composition pharmaceutique selon la revendication 8 utile dans le traitement et la prévention de la schizophrénie.

## Patentansprüche

1. Verbindungen der Formel (I): 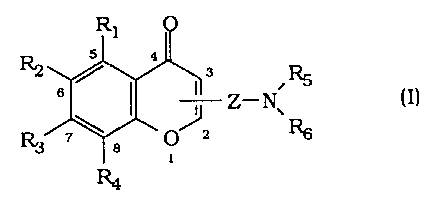 in der:
Z eine Methylenkette, die gegebenenfalls durch eine geradkettige oder verzweigte Acylgruppe substituiert ist und 1 bis 6 Kohlenstoffatome aufweist,
R₁, R₂, R₃ und R₄ unabhängig voneinander eine Gruppe ausgewählt aus:
- Wasserstoff,
- Halogen,
- Hydroxy,
- geradkettiges oder verzweigtes Alkoxy mit 1 bis 6 Kohlenstoffatomen,
- geradkettiges oder verzweigtes Alkyl mit 1 bis 6 Kohlenstoffatomen,
- Phenyl,
- Benzyl,
- Trifluormethyl,
und
R₅ und R₆ gemeinsam mit dem Stickstoffatom, das sie trägt, ein Piperidin bilden, das durch eine Phenylgruppe, Naphthylgruppe. Phenylalkylgruppe mit geradkettiger oder verzweigter Alkylgruppe mit 1 bis 6 Kohlenstoffatomen oder Naphthylalkylgruppe mit geradkettiger oder verzweigter Alkylgruppe mit 1 bis 6 Kohlenstoffatomen substituiert ist und das gegebenenfalls an den Phenyl- und Naphthylkernen durch eine oder mehrere Gruppen ausgewählt aus:
- Halogen,
- Hydroxy,
- Mercapto,
- Trifluormethyl,
- geradkettiges oder verzweigtes Alkyl mit 1 bis 6 Kohlenstoffatomen,
- geradkettiges oder verzweigtes Alkoxy mit 1 bis 6 Kohlenstoffatomen und
- geradkettiges oder verzweigtes Alkoxycarbonyl mit 1 bis 6 Kohlenstoffatomen
substituiert ist, deren optische Isomere sowie deren Additionssalze mit einer pharmazeutisch annehmbaren Säure oder Base.

2. Verbindungen der Formel (I) nach Anspruch 1, worin die Gruppe der Formel 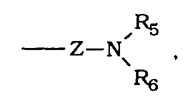 in der Z, R₅ und R₆ die in Anspruch 1 angegebenen Bedeutungen besitzen, in der 2-Stellung des Chromons in der Formel (I) nach Anspruch 1 steht, deren optische Isomere und deren Additionssalze mit einer pharmazeutisch annehmbaren Säure oder Base.

3. Verbindungen der Formel (I) nach Anspruch 1, worin die Gruppe der Formel 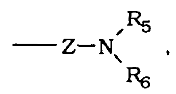 in der Z, R₅ und R₆ die in Anspruch 1 angegebenen Bedeutungen besitzen, in der 3-Stellung des Chromons in der Formel (I) nach Anspruch 1 steht, deren optische Isomere und deren Additionssalze mit einer pharmazeutisch annehmbaren Säure oder Base.

4. Verbindungen der Formel (I) nach Anspruch 1, worin Z eine Gruppe -CH₂- bedeutet, deren optische Isomere und deren Additionssalze mit einer pharmazeutisch annehmbaren Säure oder Base.

5. Verbindung nach Anspruch 1, nämlich 2-[(4-Benzylpiperidin-1-yl)-methyl]-4-oxo[4H]-1-benzopyran und dessen Additionssalze mit einer pharmazeutisch annehmbaren Säure.

6. Verfahren zur Herstellung der Verbindungen der Formel (I) nach Anspruch 1, **dadurch gekennzeichnet**, daß man ein Amin der Formel (II): 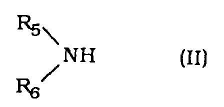 in der R₅ und R₆ die in Anspruch 1 angegebenen Bedeutungen besitzen, mit einem Halogenalkylchromon-Derivat der Formel (III): 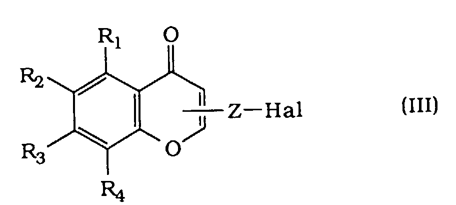 in der R₁, R₂, R₃, R₄ und Z die in Anspruch 1 angegebenen Bedeutungen besitzen und Hal ein Halogenatom darstellt,
umsetzt zur Bildung der Verbindungen der Formel (I),
welche Verbindungen der Formel (I) gewünschtenfalls
- mit Hilfe eines oder mehrerer Reinigungsverfahren ausgewählt aus Kristallisation, Chromatographie über Kieselgel, Extraktion, Filtration und Überführung über Kohlenstoff und/oder ein Harz gereinigt werden können,
- gegebenenfalls in ihre eventuellen optischen Isomeren in reiner Form oder in Form einer Mischung aufgetrennt werden können
- und/oder mit einer pharmazeutisch annehmbaren Säure oder Base in ihre Salze überführt werden können.

7. Verfahren zur Herstellung der Verbindungen der Formel (I/A): 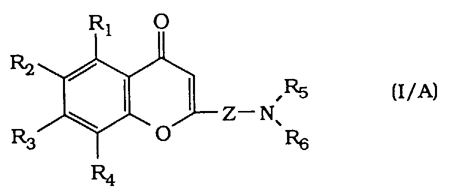 in der R₁, R₂, R₃, R₄, R₅, R₆ und Z die in Anspruch 1 angegebenen Bedeutungen besitzen, einem Sonderfall der Verbindungen der Formel (I), in der das Chromon in der 2-Stellung durch die Gruppe: 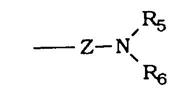 substituiert ist, **dadurch gekennzeichnet**, daß man ein Amin der Formel (II): 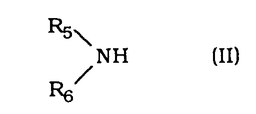 in der R₅ und R₆ die in Anspruch 1 angegebenen Bedeutungen besitzen,
mit einer Verbindung der Formel (IV):
Hal'-Z-COO-CH₂CH₃ (IV)
in der Z die oben angegebenen Bedeutungen besitzt und Hal' ein Halogenatom darstellt,
umsetzt zur Bildung einer Verbindung der Formel (V): 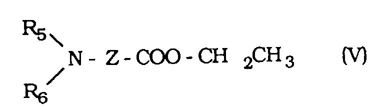 in der R₅, R₆ und Z die oben angegebenen Bedeutungen besitzen, welche Verbindung der Formel (V) man mit einem ortho-Hydroxyacetophenon der Formel (VI): 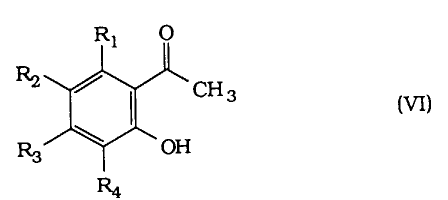 in der R₁, R₂, R₃ und R₄ die in Anspruch 1 angegebenen Bedeutungen besitzen, umsetzt zur Bildung der Verbindungen der oben definierten Formel (I/A), welche Verbindungen der Formel (I/A) gegebenenfalls
- gemäß einer oder mehrerer Reinigungsmethoden ausgewählt aus Kristallisation, Chromatographie über Kieselgel, Extraktion, Filtration und Überführung über Kohlenstoff und/oder ein Harz gereinigt werden können,
- gegebenenfalls in ihre eventuellen optischen Isomeren in reiner Form oder in Form einer Mischung aufgetrennt werden können,
- und/oder mit Hilfe einer pharmazeutisch annehmbaren Säure oder Base in ihre Salze überführt werden können.

8. Pharmazeutische Zubereitung enthaltend als Wirkstoff mindestens eine Verbindung nach Anspruch 1 in Kombination mit einem oder mehreren pharmazeutisch annehmbaren Trägermaterialien oder Bindemitteln.

9. Pharmazeutische Zubereitung nach Anspruch 8 zur Behandlung oder Vorbeugung von Streß, der Angst, von Depressionen, von Psychosen und der Schizophrenie.

10. Pharmazeutische Zubereitung nach Anspruch 8 zur Behandlung und zur Vorbeugung von Depressionen.

11. Pharmazeutische Zubereitung nach Anspruch 8 zur Behandlung und zur Vorbeugung der Schizophrenie.

## Claims

1. Compounds of formula (I) : 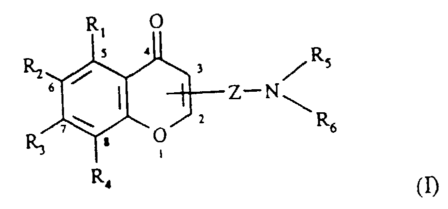 in which :
Z represents a methylene chain optionally substituted by a linear or branched acyl radical having from 1 to 6 carbon atoms,
each of R₁, R₂, R₃ and R₄, independently of the others, represents a radical selected from:
- hydrogen,
- halogen,
- hydroxy,
- linear and branched alkoxy having from 1 to 6 carbon atoms,
- linear and branched alkyl having from 1 to 6 carbon atoms,
- phenyl,
- benzyl, and
- trifluoromethyl,
R₅ and R₆ form together, with the nitrogen atom carrying them, a piperidine substituted by a phenyl radical, a naphthyl radical, a linear or branched phenylalkyl radical having from 1 to 6 carbon atoms, or a linear or branched naphthylalkyl radical having from 1 to 6 carbon atoms, optionally substituted on the phenyl and naphthyl nuclei by one or more radicals selected from:
- halogen,
- hydroxy,
- mercapto,
- trifluoromethyl,
- linear and branched alkyl having from 1 to 6 carbon atoms,
- linear and branched alkoxy having from 1 to 6 carbon atoms,
- and linear and branched alkoxycarbonyl having from 1 to 6 carbon atoms,
their optical isomers, and also their addition salts with a pharmaceutically acceptable acid or base.

2. Compounds of formula (I) according to claim 1 in which the grouping of the formula 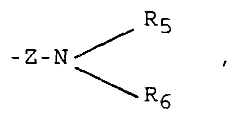 in which Z, R₅ and R₆ are as defined in claim 1, substitutes the chromone present in formula (I) according to claim 1 in the 2-position,
their optical isomers,
and their addition salts with a pharmaceutically acceptable acid or base.

3. Compounds of formula (I) according to claim 1 in which the grouping of the formula 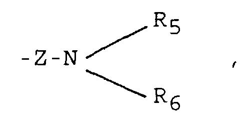 in which Z, R₅ and R₆ are as defined in claim 1, substitutes the chromone present in formula (I) according to claim 1 in the 3-position,
their optical isomers,
and their addition salts with a pharmaceutically acceptable acid or base.

4. Compounds of formula (I) according to claim 1 in which Z represents a grouping -CH₂-,
their optical isomers,
and their addition salts with a pharmaceutically acceptable acid or base.

5. Compound according to claim 1 which is 2-[(4-benzylpiperidin-1-yl)methyl]-4-oxo[4H]-1-benzopyran, and its addition salts with a pharmaceutically acceptable acid.

6. Process for the preparation of compounds of formula (I) according to claim 1, characterised in that an amine of formula (II): 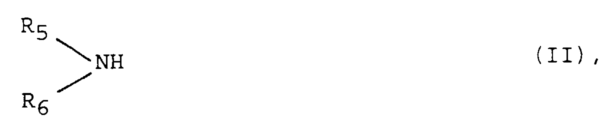 in which R₅ and R₆ are as defined in claim 1,
is reacted with a haloalkylchromone compound of formula (III): 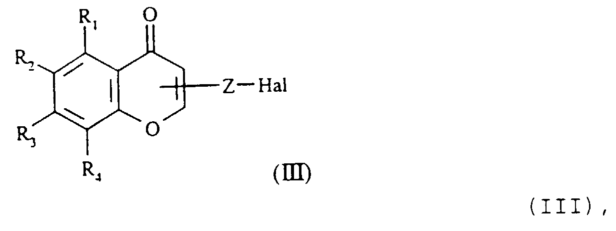 in which R₁, R₂, R₃, R₄ and Z are as defined in claim 1 and Hal represents a halogen atom,
in order to obtain the compounds of formula (I),
which compounds of formula (I) may, if desired, be
- purified in accordance with one or more purification methods selected from crystallisation, chromatography on silica gel, extraction, filtration, and passage over charcoal and/or resin,
- separated, where appropriate, in pure form or in the form of a mixture, into their possible optical isomers,
- and/or converted into a salt using a pharmaceutically acceptable acid or base.

7. Process for the preparation of compounds of formula (I/A): 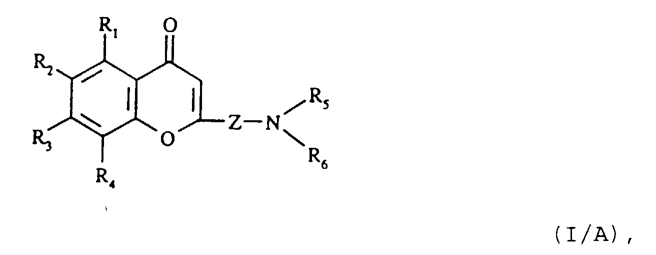 in which R₁, R₂, R₃, R₄, R₅, R₆ and Z are as defined in claim 1, which is a particular case of the compounds of formula (I) in which the chromone is substituted in the 2-position by the radical 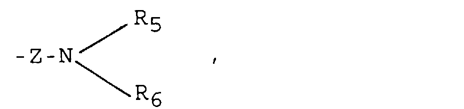 characterised in that an amine of formula (II): 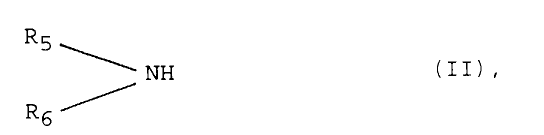 in which R₅ and R₆ are as defined in claim 1,
is reacted with a compound of formula (IV):
Hal'-Z-COO-CH₂CH₃ (IV),
in which Z is as defined above and Hal' represents a halogen atom,
in order to obtain a compound of formula (V) : 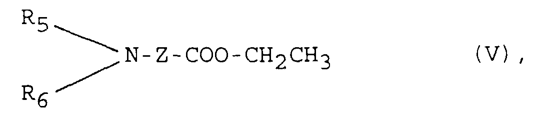 in which R₅, R₆ and Z are as defined above,
which compound of formula (V) is reacted with an ortho-hydroxyacetophenone compound of formula (VI): 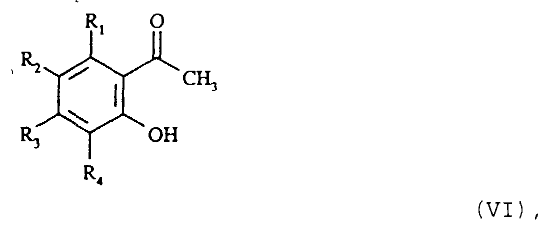 in which R₁, R₂, R₃ and R₄ are as defined in claim 1,
in order to obtain the compounds of formula (I/A) as defined above,
which compounds of formula (I/A) may, if desired, be
- purified in accordance with one or more purification methods selected from crystallisation, chromatography on silica gel, extraction, filtration, and passage over charcoal and/or resin,
- separated, where appropriate, in pure form or in the form of a mixture, into their possible optical isomers,
- and/or converted into a salt using a pharmaceutically acceptable acid or base.

8. Pharmaceutical composition containing as active ingredient at least one compound according to claim 1, in combination with one or more pharmaceutically acceptable excipients or carriers.

9. Pharmaceutical composition according to claim 8 for use in the treatment and prevention of stress, anxiety, depression, psychoses and schizophrenia.

10. Pharmaceutical composition according to claim 8 for use in the treatment and prevention of depression.

11. Pharmaceutical composition according to claim 8 for use in the treatment and prevention of schizophrenia.
